# EUROPÄISCHE PATENTSCHRIFT

(11) **EP 0 805 634 B1**
(45) Veröffentlichungstag und Bekanntmachung des Hinweises auf die Patenterteilung: **18.11.1998**
(21) Anmeldenummer: 96900874.7
(22) Anmeldetag: 26.01.1996
(51) Int. Cl.: A43D 1/02

(54) **FUSSGRÖSSENMESSGERÄT**
FOOT-MEASURING DEVICE
DISPOSITIF DE MESURE DU PIED

(30) Priorität: 26.01.1995 DE 29501215 U
(43) Veröffentlichungstag der Anmeldung: 12.11.1997
(73) Patentinhaber: Hauptverband der Deutschen Schuhindustrie e.V., 63065 Offenbach (DE)
(72) Erfinder: KNIPPING, Lebrecht, D-58762 Altena (DE)
(74) Vertreter: Rasch, Michael
(86) Internationale Anmeldenummer: DE9600134
(87) Internationale Veröffentlichungsnummer: WO9622713

(56) Entgegenhaltungen:
- EP-A- 0 005 038
- DE-A- 2 447 474

## Beschreibung

Die vorliegende Erfindung betrifft ein Fußgrößenmeßgerät zur Messung der Fußgröße, insbesondere der Fußlänge und -breite, gemäß dem Oberbegriff des Anspruchs 1. Ein solches Gerät ist aus der DE-A-24 47 474 bekannt.

Derartige Geräte sind bekannt, um insbesondere beim Kaufvon Schuhen die genaue Größe des Fußes einer Person zu ermitteln und damit die Auswahl geeigneter Schuhe so einfach wie möglich zu gestalten. Insbesondere bei Kleinhindern stellt sich das Problern, daß es wegen deren O-förmig gebogener Beine mit nach innen gedrehten Füßen schwierig ist, die Kinderfüße auf herkömmliche Fußgrößenmeßgeräte zu stellen und ein genaues Ablesen der Fußgröße zu ermöglichen.

Obwohl die Fußgröße vorzugsweise im Stehen gemessen werden sollte, ist dies bei kleinen Kindern häufig nicht oder nur schwer möglich, so daß das Fußgrößenmeßgerät in diesem Fall bei sitzender oder liegender Stellung der zu vermessenden Person von Benutzer des Gerätes gegen den Fuß gedrückt werden müßte. Dieser Vorgang ist bei Verwendung der herkömmlichen Fußgrößenmeßgeräten schwierig, erfordert die volle Aufmerksamkeit des Benutzers sowie beide Hände zur Handhabung des Gerätes, insbesondere weil das Fußgrößenmeßgerät zur Vermeidung von Meßverfälschungen gegen die Fußsohle gedrückt werden sollte.

Dieses Problem kann ferner bei Verwendung der sogenannten "WMS"-Meßskala in Erscheinung treten, bei der nicht nur die Länge des Fußes sondern auch dessen Breite in drei Bereichen weit, mittel, schmal, bestimmt wird, um eine noch präziserer Anpassung geeigneter Schuhe vorzunehmen.

Daher liegt der Erfindung die Aufgabe zugrunde, ein gattungsgemäßes Fußgrößenmeßgerät anzugeben, das in der Handhabung und Bedienung besonders einfach ist und gleichzeitig eine genaue Feststellung der Fußgröße ermöglicht.

Erfindungsgemäß wird diese Aufgabe durch die im Anspruch 1 angegebenen Merkmale gelöst. Die Unteransprüche beinhalten zweckmäßige Weiterbildungen.

Die Erfindung ermöglicht eine sehr bequeme Feststellung der Länge und Breite des zu vermessenden Fußes, da das Gerät mit einer Hand gehalten und gleichzeitig mittels Daumen oder Finger durch Verschiebung des Anschlages und des Seitenführungsschiebers eingestellt werden kann. Durch den Seitenführungschieber wird zum einen eine Ausrichtung des Fußes parallel zur Verschieberichtung des längsverschieblichen Anschlages erreicht und zum anderen kann die Fußbreite an einer geeigneten Anzeigeeinrichtung, vorzugsweise in der "WMS"-Skala abgelesen werden. Gleichzeitig kann die Fußlänge in der gewählten Größenskala abgelesen werden, wenn der Anschlag in Berührung mit dem vordersten Zehe gebracht ist.

Aufgrund der Tatsache, daß das erfindungsgemäße Fußgrößenmeßgerät eine Haltestütze zum Halten des Gerätes mit einer Hand aufweist und das Eingriffselement zur gemeinsamen manuellen Betätigung des verschiebbaren Anschlags und des Seitenführungsschiebers vorzugsweise durch den Daumen oder einen Finger der gleichen Hand betätigt werden kann, hat der Benutzer die weite Hand frei, beispielsweise um die oben erwähnte Anpresskraft gegen die Fußsohle zu erzeugen. Durch die Erfindung ergibt sich insgesamt eine erhebliche Vereinfachung und Erleichterung des Meßvorgangs zur Bestimmung der Fußgröße.

Zweckmäßigerweise ist das Eingriffselement als Mulde ausgebildet: die in einem Führungsteil des Seitenführungsschiebers angeordnet ist, wodurch dieser Seitenführungsschieber unmittelbar in Querrichtung feststelltwerden kann. Aufgrund der Tatsache, daß der Seitenführungsschieber am längsverschieblichen Anschlag angebracht ist, läßt sich gleichzeitig durch Einwirken auf das muldenartige Eingriffselement der Anschlag zum Messen der Fußlänge in seiner Bewegungsrichtung verschieben.

Vorzugsweise ist der verschiebbare Anschlag mittels einer Kugelführung oder einer Rollenführung an der Grundplatte geführt, wodurch sich im Betrieb eine Verstellung unter geringem Kraftaufwand möglich ist und auch die Gefahr einer Verklemmung weitgehend vermieden werden kann.

Zusätzlich ist es möglich, im Bereich des Eingriffselements eine Klemmeinrichtung vorzusehen, die den längsverschieblichen Anschlag sowie den Seitenführungsschieber so lange in ihrem gegenwärtigen Stellungen festgeklemmt halten, bis die Klemmeinrichtung durch Einwirken auf das Eingriffselement gelöst wird und eine Verschiebung sowohl des Anschlags als auch des Seitenführungsschiebers möglich wird. Befinden sich dann sowohl der verschiebbare Anschlag als auch der Seitenführungsschieber in Berührung mit dem zu vermessenden Fuß, wird das Eingriffselement losgelassen und die Klemmeinrichtung fixiert Anschlag und Seitenführungsschieber in dieser Stellung, wodurch die Ablesung des Meßergebnisses erleichtert wird.

Vorzugsweise ist der verschiebbarer Anschlag im wesentlichen L-förmig ausgebildet und weist eine dem Seitenführungsschieber gegenüberliegende Seitenplatte auf Die Seitenplatte dient zum einen als dem Seitenführungsschieber gegenüberliegende Seitenführung, zwischen denen die Fußbreite gemessen wird. Zum anderen können zweckmäßigerweise am grundplattenseitigen Rand der Seitenplatte die oben erwähnten Führungseinrichtungen zum Verschieben des Anschlages angebracht sein.

Die Haltestütze zum Halten des Gerätes ist vorzugsweise als in Längsrichtung an der Rückseite der Grundplatte angeordnete, etwa quarderförmige Stützschiene ausgebildet, die vorzugsweise eine Breite und Höhe von ca. 1 bis 2 cm aufweist sowie eine Länge, die ein Hintergreifen mit allen Fingern einer Hand ermöglicht. Zweckmäßigerweise ist die Stützschiene zumindest teilweise mit einer Riffelung oder einer anderen geeigneten Oberflächenstruktur versehen.

Die Erfindung wird nachfolgend anhand der beigefügten Zeichnungen weiter erläutert. Dabei zeigt:
- Figur 1: eine Seitenansicht des erfindungsgemäßen Fußgrößenmeßgeräts,
- Figur 2: eine Draufsicht des Gerätes,
- Figur 3: einen Schnitt entlang der Linie III-III von Figur 2, und
- Figur 4: eine perspektivische Ansicht der Rückseite des Gerätes.

Das erfindungsgemäße Fußgrößenmeßgerät besteht im wesentlichen aus einer Grundplatte 10, auf deren Oberseite eine etwa L-förmige Fersenstützplatte 12 angeformt ist. Ein verschiebbarer Anschlag 14, der mit einer Seitenplatte 16 verbunden ist, ist über eine Rollenführung 18 entlang der Grundplatte 10 verschiebbar, wobei die Seitenplatte 16 durch einen Längsschlitz 20 in der Grundplatte 10 hindurchragt. Am Anschlag 14 ist ferner ein Anzeiger 22 angeformt, der mit einer an der Grundplatte 10 angebrachten Skala 24 zur beige der gemessenen Fußgröße dient. Im dargestellten Fall ist das Fußgrößenmeßgerät zur Messung von Kinderfüßen eingerichtet und weist daher eine Skala der Schuhgrößen 17 bis 25 auf. Selbstverständlich kann das Gerät durch Verlängerung in Längsrichtung auch zur Messung größerer Füße ausgebildet sein.

An dem in Längsrichtung verschiebbaren Anschlag 14 ist ein in dessen Erstreckungsrichtung, das heißt senkrecht zu Bewegungsrichtung des Anschlags 14, verschiebbarer Seitenführungsschieber 26 angebracht. Dieser umfaßt ein Fuhrungsteil 28 mit einem muldenartigen Eingriffselement 30 sowie einen Anzeiger 32 zur Anzeige der Fußbreite auf einer an der Grundplatte 10 angebrachten Breitenskala 34.

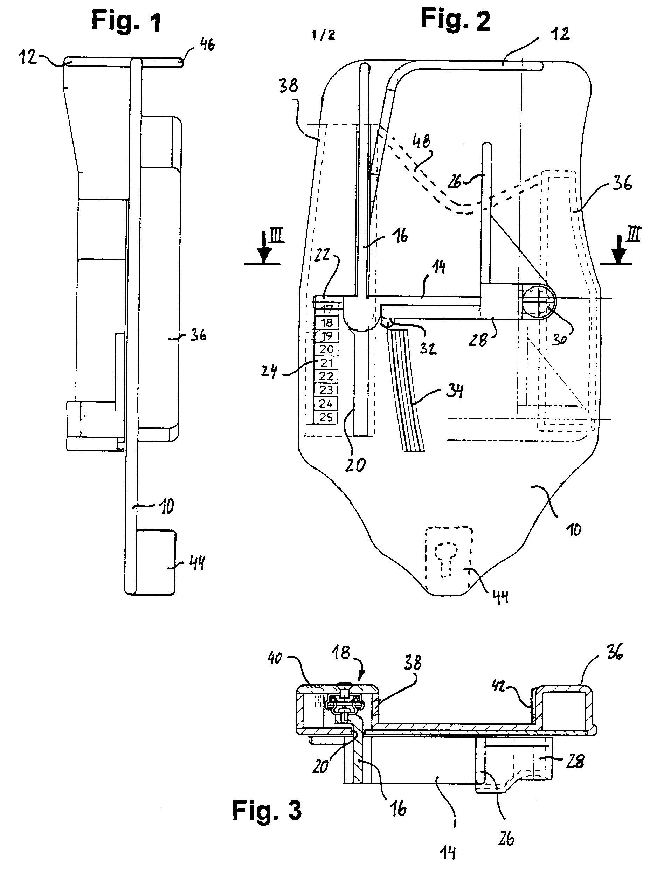

In der in Figur 2 dargestellten Stellung befinden sich der Anschlag 14 sowie der Seitenführungsschieber 26 jeweils in ihrer Endstellung für den kleinsten bzw. schmalsten Fuß. Weiter rechts unten in Figur 2 ist die entgegengesetzte Endstellung für den längsten bzw. breitesten Fuß gestrichelt dargestellt.

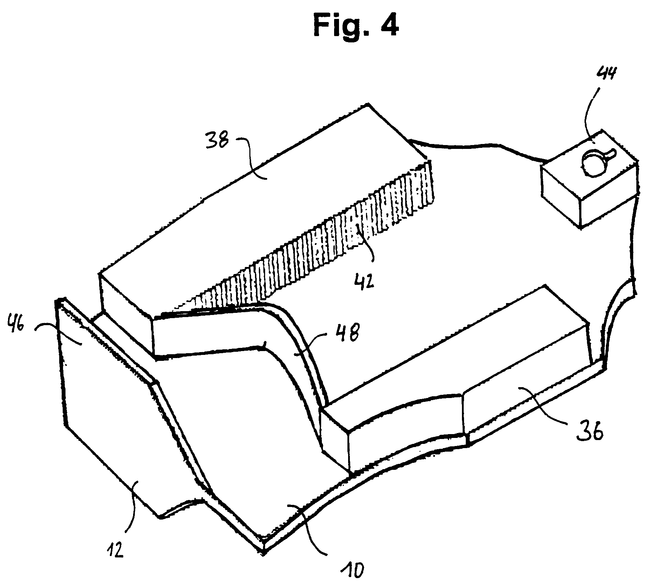

Wie am besten in Figur 4, sowie gestrichelt in Figur 2 zu erkennen ist, ist an der Rückseite der Grundplatte 10 eine Haltestütze 36 angeformt, die zum Hintergreifen durch die Finger beim Fassen des Gerätes dient. Auf der gegenüberliegenden Seite ist an der Rückseite der Grundplatte 10 ein Gehäuse 38 für die Rollenführung 18 vorgesehen, die vorzugsweise einen in Figur 3 erkennbaren abnehmbaren Deckel 40 umfaßt.

Die sich gegenüberliegenden, nach innen gerichteten Seiten der Haltestütze 36 und/oder des Gehäuses 38 können Riffelungen 42 zur Erhöhung der Griffsicherheit umfassen.

Am einen Längsende ist an der Rückseite der Grundplatte 10 ferner eine Aufhängeeinrichtung 44 mit einer schlüssellochartigen Öffnung zum Einhängen des Gerätes in einen Befestigungshaken o.ä. angeformt. Diese Aufhängeeinrichtung 44 weist vorzugsweise die gleiche Erstreckung senkrecht zur Grundplatte 10 wie die Haltestütze 36 und das Gehäuse 38 auf, um eine stabile Abstützung des Gerätes zu erreichen, wenn dieses auf den Boden gestellt wird.

Am gegenüberliegenden Ende der Grundplatte 10 ist an dessen Rückseite ferner eine Stützplatte 46 angeordnet. Diese bildet vorzugsweise zusammen mit der in die entgegengesetzte Richtung abstehender Fersenstützplatte 12 eine zusammenhängende Fläche, mittels der das Fußgrößenmeßgerät beispielsweise vertikal aufgestellt werden kann. Diese Stützplatte 46 dient vornehmlich dazu, das Fußgrößenmeßgerät sicher und stabil auf dem Boden zu plazieren, wenn sich eine Person mit ihrem Gewicht auf das Gerät stellt, da das Hauptgewicht vornehmlich im Fersenbereich, das heißt im Bereich der Fersenstützplatte 12, nach unten zu übertragen ist. Vorzugsweise liegen die Haltestütze 36, das Gehäuse 38, die Aufhängeeinrichtung 44 und die Stützplatte 46 mit ihren freien Endflächen in der gleichen Ebene, damit das Gerät eben an die Wand gehängt oder aufden Boden gestellt werden kann.

An der Rückseite der Grundplatte 10 ist ferner zwischen der Haltestütze 36 und dem Gehäuse 38 eine etwa V-förmige Halteleiste 48 vorgesehen, die dazu dient, den Fingern der zweiten Hand des Benutzers zum Andrücken des Meßgerätes an den Kinderfüß besseren Halt zu geben.

Bei der Benutzung wird das erfindungsgemäße Fußgrößenmeßgerät dadurch gehalten, daß ein Benutzer mit seiner rechten Hand die Haltestütze umgreift und mit dem Daumen in die Mulde 30 eingreift. Mittels des Daumens läßt sich dann sowohl der Anschlag 14 in seiner Bewegungsrichtung als auch der Seitenführungsschieber 26 in seiner dazu senkrechten Bewegungsrichtung verstellen und in Berührung mit einem zu vermessenen Fuß bringen. Um die Meßgenauigkeit zu erhöhen, kann mit der anderen Hand unter Umfassung der V-förmigen Griffleiste 48 der Fersenbereich des Fußgrößenmeßgerätes von unten gegen die Ferse gedrückt werden. Die Breite des Fußes läßt sich sodann an der Breitenskala 34 und die Länge an der Skala 24 ablesen. Diese Skalen 34 und 24 sind vorzugsweise in der "WMS"-Norm skaliert.

Die Randkontur der Grundplatte 10 weist in der gezeigten Ausführungsform eine unregelmäßige Kontur auf was darauf zurückzuführen ist, daß im Hinblick auf die Vermessung von Kinderfüßen eine Abbildung, beispielsweise eines Clowns, darauf angebracht werden kann. Die bogenförmig nach innen gekrümmte Ausbildung des Plattenrandes im Bereich der Haltestütze 36 sowie die daran angepaßte Krümmung der Haltestütze 36 selbst dient zur Anlage des Daumens, wenn dieser nicht gegen die Mulde 30 drückt.

Das erfindungsgemäße Fußgrößenmeßgerät zeichnet sich durch eine besonders leichte Handhabung aus, was insbesondere bei der Vermessung von Kinderfüßen zweckmäßig ist. Das Gerät ist leichtgängig und insbesondere mit einer Hand zu bedienen, falls die zweite Hand zum Halten oder zum Erzeugen von Druck gegen den Fuß benötigt wird.

Die Messung der Fußgröße sollte, soweit wie möglich, im Stehen erfolgen, damit das volle Körpergewicht auf den Füßen lastet. Wenn nun Füße kleiner im Sitzen, beispielsweise auf den Schoß der Mutter, gemessen werden sollen, muß der fehlende Druck auf die Füße des Kindes durch den Benutzer des Meßgerätes erzeugt werden. Durch die erfindungsgemäße Ausbildung der Rückseite des Gerätes mit verschiedenen Griffleisten 36, 38, 48 ist ein individuelles Festhalten des Gerätes je nach Situation und Handgröße des Benutzers möglich. Das Gerät kann auch einhändig an der Haltestütze 36 gepackt werden, wobei der Anschlag 14 und der Seitenführungsschieber 26 durch Betätigung der Daumenmulde 30 bewegt werden können.

## Patentansprüche

1. Fußgrößenmeßgerät mit einer Grundplatte (10), einem ersten festen Anschlag (12) sowie einem diesem gegenüberliegenden, verschiebbaren zweiten Anschlag (14), der mit einer Einrichtung (22, 24) zur beige der gemessenen Fußlänge gekoppelt ist, und am verschiebbaren Anschlag (14) ein quer zu dessen Bewegungsrichtung verschiebbarer Seitenführungsschieber (26) angebracht ist, der mit einer Einrichtung (32, 34) zur Anzeige der Fußbreite gekoppelt ist, dadurch gekennzeichnet, daß eine Haltestutze (36) zum Halten des Gerätes mit einer Hand sowie ein Eingriffselement (30) zur gemeinsamen manuellen Betätigung des verschiebbaren Anschlags (14) und des Seitenführungsschiebers (26) mittels der gleichen Hand vorgesehen sind.

2. Fußgrößenmeßgerat nach Anspruch 1, dadurch gekennzeichnet, daß das Eingriffselement als Mulde (30) ausgebildet ist

3. Fußgrößenmeßgerät nach Anspruch 1 oder 2 dadurch gekennzeichnet, daß der verschiebbare Anschlag (14) mittels einer Kugel- oder Rollenführung (18) an der Grundplatte (10) geführt ist.

4. Fußgrößenmeßgerät nach Anspruch 3, dadurch gekennzeichnet, daß der verschiebbare Anschlag (14) im wesentlichen L-förmig ausgebildet ist und eine dem Seitenführungsschieber (26) gegenüberliegende Seitenplatte (16) aufweist.

5. Fußgrößenmeßgerät nach einem der vorhergehenden Ansprüche, dadurch gekennzeichnet, daß die Stützschiene (36) zumindest bereichsweise mit einer Riffelung (42) versehen ist.

6. Fußgrößenmeßgerät nach Anspruch 3, dadurch gekennzeichnet, daß die Rollenführung (18) für den verschiebbaren Anschlag (14) an der Rückseite der Grundplatte (10) angeordnet und von einem Gehäuse (38) umschlossen ist.

7. Fußgrößenmeßgerät nach Anspruch 6, dadurch gekennzeichnet, daß die Stützschiene (36) und das Rollenführungsgehäuse (38) im wesentlichen parallel zueinander an der Rückseite der Grundplatte (10) angeordnet sind und mittels einer etwa unterhalb des festen Anschlags (12) liegenden Halteleiste (48) miteinander verbunden sind.

8. Fußgrößenmeßgerät nach Anspruch 7, dadurch gekennzeichnet, daß die Halteleiste (48) etwa V-förmig ist.

9. Fußgrößenmeßgerät nach einem der vorhergehenden Ansprüche, dadurch gekennzeichnet, daß es zur Messung von Füßen der Größe 15 bis 30 ausgebildet ist.

10. Fußgrößenmeßgerät nach einem der vorhergehenden Ansprüche, dadurch gekennzeichnet, daß die Fußbreite in mindestens drei Bereichen schmal, mittel und weit, angegeben ist.

## Claims

1. Foot-size measuring apparatus, having a base plate (10), a first fixed stop member (12) and a displaceable second stop member (14), which is situated opposite the first stop member and is connected to an arrangement (22, 24) for indicating the measured foot length, and a lateral guidance slide (26) is mounted on the displaceable stop member (14), said slide being displaceable transversely relative to the direction of movement of the displaceable stop member and being connected to an arrangement (32, 34) for indicating the foot width, characterised in that a retaining support (36) for retaining the apparatus with one hand and an engagement element (30) for the joint manual actuation of the displaceable stop member (14) and of the lateral guidance slide (26) by means of the same hand are provided.

2. Foot-size measuring apparatus according to claim 1, characterised in that the engagement element is configured as depression (30).

3. Foot-size measuring apparatus according to claim 1 or 2, characterised in that the displaceable stop member (14) is guided on the base plate (10) by means of a ball or roller guide (18).

4. Foot-size measuring apparatus according to claim 3, characterised in that the displaceable stop member (14) has a substantially L-shaped configuration and a lateral plate (16), which is situated opposite the lateral guidance slide (26).

5. Foot-size measuring apparatus according to one of the preceding claims, characterised in that the supporting bar (36) is provided with a corrugation (42) in various regions at least.

6. Foot-size measuring apparatus according to claim 3, characterised in that the roller guide (18) for the displaceable stop member (14) is disposed on the rear side of the base plate (10) and surrounded by a housing (38).

7. Foot-size measuring apparatus according to claim 6, characterised in that the supporting bar (36) and the roller guide housing (38) are disposed substantially parallel to each other on the rear side of the base plate (10) and are interconnected by means of a retaining strip (48), which lies roughly beneath the fixed stop member (12).

8. Foot-size measuring apparatus according to claim 7, characterised in that the retaining strip (48) is roughly V-shaped.

9. Foot-size measuring apparatus according to one of the preceding claims, characterised in that it is configured to measure feet between sizes 15 and 30.

10. Foot-size measuring apparatus according to one of the preceding claims, characterised in that the foot width is indicated in at least three ranges: narrow, medium and wide.

## Revendications

1. Instrument de mesure de la taille d'un pied, comportant une plaque de base (10), une première butée fixe (12) ainsi que, placée en face de cette dernière, une seconde butée coulissante (14), qui est couplée à un dispositif (22, 24) pour l'indication de la longueur mesurée du pied, tandis que sur la butée coulissante (14), est montée, déplaçable transversalement à la direction de déplacement de cette dernière, une pièce coulissante de guidage latéral (26), qui est couplée à un dispositif (32, 34) pour l'indication de la largeur du pied, caractérisé en ce qu'il est prévu un talon de soutien (36), permettant de tenir l'instrument avec une main, ainsi qu'un élément de prise (30) permettant de manoeuvrer manuellement ensemble la butée coulissante (14) et la pièce coulissante de guidage latéral (26) à l'aide de la même main.

2. Instrument de mesure de la taille d'un pied selon la revendication 1, caractérisé en ce que l'élément de prise est réalisé sous forme d'une cavité (30).

3. Instrument de mesure de la taille d'un pied selon la revendication 1 ou 2 caractérisé en ce que la butée coulissante (14) est guidée, sur la plaque de base (10), au moyen d'un ensemble de guidage à billes ou à galets (18).

4. Instrument de mesure de la taille d'un pied selon la revendication 3, caractérisé en ce que la butée coulissante (14) est réalisée sous une forme sensiblement en L et comporte une plaquette latérale (16) placée en face de la pièce coulissante de guidage latéral (26).

5. Instrument de mesure de la taille d'un pied selon l'une quelconque des revendications 1 à 4, caractérisé en ce que la barre d'appui (36) est pourvue, au moins par endroits, d'un striage (42).

6. Instrument de mesure de la taille d'un pied selon la revendication 3, caractérisé en ce que l'ensemble de guidage à galets (18) pour la butée coulissante (14) est disposé au dos de la plaque de base (10) et est entouré par un carter (38).

7. Instrument de mesure de la taille d'un pied selon la revendication 6, caractérisé en ce que la barre d'appui (36) et le carter (38) de l'ensemble de guidage à galets sont disposés de façon à être sensiblement parallèles l'un à l'autre, au dos de la plaque de base (10), et sont reliés l'un à l'autre au moyen d'une nervure de soutien (48) située à peu près au-dessous de la butée fixe (12).

8. Instrument de mesure de la taille d'un pied selon la revendication 7, caractérisé en ce que la nervure de soutien (48) est approximativement en forme de V.

9. Instrument de mesure de la taille d'un pied selon l'une qelconque des revendications 1 à 8, caractérisé en ce qu'il est conçu pour la mesure de pieds de taille 15 à 30.

10. Instrument de mesure de la taille d'un pied selon l'une quelconque des revendications 1 à 9, caractérisé en ce que la largeur du pied est donnée en au moins trois plages, petite, moyenne et grande.
